(19) Europäisches Patentamt
European Patent Office
Office européen des brevets

(11) EP 3 370 735 B1

(12) EUROPEAN PATENT SPECIFICATION

(45) Date of publication and mention of the grant of the patent:
**13.05.2020 Bulletin 2020/20**

(21) Application number: **16804685.2**

(22) Date of filing: **31.10.2016**

(51) Int Cl.:
***A61K 31/717*** *(2006.01)* ***A61K 31/722*** *(2006.01)*
***A61K 31/738*** *(2006.01)*

(86) International application number:
**PCT/CZ2016/050039**

(87) International publication number:
**WO 2017/076378 (11.05.2017 Gazette 2017/19)**

# (54) POLYSACCHARIDE-BASED POLYMER AND ITS USE FOR THE PREVENTION AND TREATMENT OF THE WILSON'S DISEASE

POLYSACCHARIDBASIERTES POLYMER UND DESSEN VERWENDUNG ZUR PRÄVENTION UND BEHANDLUNG DER WILSON-KRANKHEIT

POLYMÈRE À BASE DE POLYSACCHARIDES ET SON UTILISATION EN VUE DE LA PRÉVENTION ET DU TRAITEMENT DE LA MALADIE DE WILSON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.11.2015 CZ 20150770**

(43) Date of publication of application:
**12.09.2018 Bulletin 2018/37**

(73) Proprietors:
- **Ustav Makromolekularni Chemie AV CR, v.v.i.**
**162 06 Praha 6 (CZ)**
- **Univerzita Karlova V Praze, 1. Lekarska Fakulta**
**12108 Praha 2 (CZ)**

(72) Inventors:
- **HRUBY, Martin**
**16000 Praha 6 (CZ)**
- **VETRIK, Miroslav**
**01864 Koseca (SK)**
- **KUCKA, Jan**
**25265 Holubice-Kozinec (CZ)**
- **MACKOVA, Hana**
**46006 Liberec 6 (CZ)**
- **STEPANEK, Petr**
**15000 Praha 5 (CZ)**
- **HUMAJOVÁ, Jana**
**190 00 Praha 9 (CZ)**
- **POUCKOVA, Pavla**
**25229 Dobrichovice (CZ)**
- **URBANEK, Petr**
**15000 Praha 5 (CZ)**

(74) Representative: **Hartvichova, Katerina**
**HARBER IP s.r.o.**
**Dukelskych hrdinu 567/52**
**170 00 Praha 7 (CZ)**

(56) References cited:
**WO-A1-02/085383**

- **HUSTER, D.: "Wilson disease", BEST PRACTICE & RESEARCH IN CLINICAL GASTROENTEROLOGY, vol. 24, 2010, pages 531-539, XP002767082, cited in the application**
- **ALA, A.; WALKER, A. P.; ASHKAN, K.; DOOLEY, J. S.; SCHILSKY, M. L.: "Wilson's disease", LANCET, vol. 369, 2007, pages 397-408, XP002767083, cited in the application**
- **VIBHA GURNANI ET AL: "Cellulose functionalized with 8-hydroxyquinoline: new method of synthesis and applications as a solid phase extractant in the determination of metal ions by flame atomic absorption spectrometry", ANALYTICA CHIMICA ACTA, vol. 485, no. 2, 1 June 2003 (2003-06-01), pages 221-232, XP055344350, AMSTERDAM, NL ISSN: 0003-2670, DOI: 10.1016/S0003-2670(03)00416-1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 370 735 B1

## Description

### Field of Art

The invention relates to the polymers bonding copper ions suitable for peroral administration for the prevention and mitigation of consequences of Wilson's disease.

### Background Art

Wilson's disease is a genetically determined disease characterized by a disorder of copper metabolism leading to cirrhosis of the liver and degeneration of the basal ganglia. [Ala, A., Walker, A. P., Ashkan, K., Dooley, J. S., Schilsky, M. L. (2007), Wilson's disease. Lancet 369, 397-408]. The cause of the disease is a malfunction of the enzyme ATPase 7B caused by a mutation of a particular gene [Bruha, R., Marek, Z., Pospíšilová, L., Nevšímalová, S., Vítek, L., Martásek, P., Nevoral, J., Petrtyl, J., Urbánek, P., Jirásková, A., Ferenci, P. (2010) Long-term follow-up of Wilson's Disease: natural history, treatment, mutations analysis and phenotypic correlation. Liver International 31, 83-91], currently there are more than 400 mutations of this gene described. Average prevalence in the population is 1 : 30 000. Malfunction of the ATPase 7B significantly reduces copper excretion into the bile, which is the major route of elimination of copper from the body in humans. This then leads to accumulation of copper in the body, especially the liver and in the central nervous system. High concentrations of copper in tissues also leads to a number of symptoms due to toxic oxidative stress, including in particular damage to the liver, brain, and parenchymal organs. Symptoms usually first manifest in older children and adolescents. Typical clinical symptoms are three: liver damage (most often), neurological problems, and psychosis. If the Wilson's disease is not treated, it gradually leads to death of the patient. The most serious complications in patients with hepatic damage are liver cirrhosis, portal hypertension, and massive gastrointestinal bleeding.

First line therapy is currently based on reducing the amount of copper in the body by the administration of low-molecular-weight chelators of copper (penicillamine, triethylenetetramine or tetrathiomolybdate), which leads to reduced absorption of copper from the diet and reabsorption of copper excreted into the digestive tract and its increased urinary excretion. Maintenance therapy involves particularly administration of high doses of zinc(II) salts as zinc ions block the intake of copper from the gastrointestinal tract. Unfortunately, suitable forms of zinc are not available in all countries.

The use of these drugs is however limited by multiple and often serious side effects such as for example myelosuppression, lupus and myasthenia of penicillamine [Huster, D. (2010) Wilson disease. Best Practice & Research in Clinical Gastroenterology 24, 531-539; Das, S. K., Ray, K. (2006) Wilson's disease: an update. Nature Clinical Practice Neurology 2, 482-493], which is a consequence of re-complexing of the essential elements in the body after resorption of the chelating agent from the gastrointestinal tract. Treatment with zinc(II) salts is also typically accompanied by severe gastrointestinal symptoms (the doses of zinc used, up to 1200 mg per day [Brewer, G. J., Yuzbasiyan-Gurkan, V., Johnson, V., Dick, R.D., Wang, Y (1993) Treatment of Wilson's Disease with Zinc XII: Dose Regimen Requirements. The American Journal of the Medical Sciences 305, 199-202], represent approximately one hundred times the normal daily intake, which is about 8 - 15 mg, in fact, causing a slight zinc intoxication).

Dietary intake of copper is 0.6 to 1.6 mg per day, so that as adjuvant therapy of Wilson's disease is recommended - at the beginning of therapy - a diet with low content of copper, i.e. omitting the food with its high content, such as liver, nuts or mushrooms (due to the ubiquity of the copper in food, however, a diet without copper is impossible).

A significant amount of copper is excreted in the digestive tract and then again resorbed, this amount represents an even greater amount than in the diet (daily is secreted about 4.4 to 5.3 mg of copper, of which 7 % in saliva, 20 % of gastric juice, 50 % in the bile, 18 % in pancreatic juice and 5 % in duodenal secretions) [van den Berghe, P. V. E., Klomp, L. W. J. (2009) New developments in the regulation of intestinal copper absorption. Nutrition Reviews 67, 658-672].

The objective of the submitted invention is to provide a preparation for peroral use for the prevention and mitigation of consequences of Wilson's disease, which will selectively adsorb copper in the digestive tract only and will not exhibit undesirable side effects.

### Disclosure of Invention

The present invention provides polymer particles containing functional groups chelating copper(II) ions, that have all properties necessary for peroral use for the prevention and mitigation of consequences of the Wilson's disease and features allowing this use.

The subject of the present invention is a polymer containing monomeric units of a general formula I

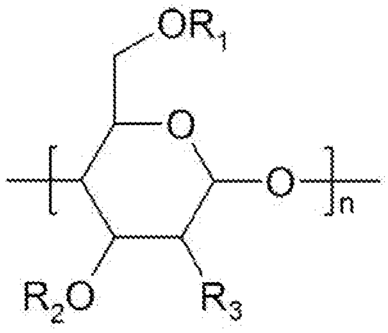

(I)

wherein

$R_1$ is selected from a group containing H, $-CH_2-CH(OH)-CH_2-NR_4R_5$;
$R_2$ is selected from a group containing H, $-CH_2-CH(OH)-CH_2-NR_4R_5$;
$R_3$ is selected from a group containing OH, $-O-CH_1-CH(OH)-CH_1-NR_4R_5$, $NR_4R_5$;
n is an integer in the range of from 60 to 50 000;
$R_4$ is selected from a group containing H, $(C_1-C_6)$alkyl, hydroxy$(C_1-C_6)$alkyl, $-CH_2$-[8-hydroxyquinoline], $-CH_2$-[8-hydroxyquinoline-5-sulfonic acid], 2-pyridylmethyl;
if $R_3$ is $NR_4R_5$, then from 0 to 50 mol. % of substituents $R_4$ in this group may be acetyl;
$R_5$ is selected from a group containing H, $(C_1-C_6)$alkyl, hydroxy$(C_1-C_6)$alkyl, $-CH_2$-[8-hydroxyquinoline], $-CH_2$-[8-hydroxyquinoline-5-sulfonic acid], 2-pyridylmethyl;
wherein at leas 5 mol. % of the total amount of substituents $R_1$, $R_2$, and $R_3$ in the polymer carry the substituent $NR_4R_5$, wherein $R_4$ and/or $R_5$ is a group selected from the group containing $-CH_2$-[8-hydroxyquinoline], $-CH_2$-[8-hydroxyquinoline-5-sulfonic acid], 2-pyridylmethyl;
and wherein the polymer is cross-linked so that at least 0.1 mol. % of monomeric units are covalently bridged.

Covalent bridging of monomeric units is in one preferred embodiment done through the following bridges:

- in case that $R_3$ is $NR_4R_5$, then substituents $R_4$ and/or $R_5$ present in this group of two monomeric units form together the bridge $=C-(CH_2)_n-C=$, where n is from 0 to 8, or $-CH_2-CH(OH)-CH_2-$;
- in case that $R_3$ is not $NR_4R_5$, then $R_3 = OR_3'$, and one or more of the substituents $R_1$, $R_2$, $R_3'$ of one monomeric unit form(s) together with $R_1$, $R_2$ or $R_3'$ of another unit a bridge with the structure of $-CH_2-CH(OH)-CH_2-N(R_6)-CH_2-CH(OH)-CH_2-$, where $R_6$ is selected from a group containing H, $(C_1-C_6)$alkyl, hydroxy$(C_1-C_6)$alkyl, $-CH_2$-[8-hydroxyquinoline],$-CH_2$-[8-hydroxyquinoline-5-sulfonic acid], 2-pyridylmethyl.

In one preferred embodiment, the cross-linkage of the polymer is such that at least 1 mol. % of monomeric units are covalently bridged, preferably maximum 50 mol. % of monomeric units are covalently bridged, more preferably maximum 30 mol. % of monomeric units are covalently bridged. Most preferably, from 3 to 10 mol. % of monomeric units are covalently bridged, optimally 5 mol. % of monomeric units are covalently bridged.

In one preferred embodiment, the monomeric units are monomeric units of cellulose, functionalized by the substituents $R_1$, $R_2$, $R_3$, as defined above (when $R_3$ is not $NR_4R_5$). In another preferred embodiment, the monomeric units are monomeric units of chitosan, functionalized with the substituents $R_1$, $R_2$, $R_3$, as defined above (when $R_3$ is $NR_4R_5$).

If cellulose or chitosan are used for the synthesis, the initial polysaccharide has in a preferred embodiment its average molecular weight of from 10 000 to 8 000 000 g/mole, preferably from 20 000 to 60 000 g/mole.

Preferably, the polymer is in a form of microparticles of sizes from 1 to 1 000 micrometers.

The chitosan has a degree of deacetylation from 50 to 100 % (i.e. the maximum of 50 mol. % of $R_4$ in the group $R_3=NR_4R_5$ is acetyl), preferably from 75 to 85 % (i.e. from 15 to 25 mol. % $R_4$ in the group $R_3=NR_4R_5$ is acetyl).

The polymer is then on the basis of chemically cross-linked biopolymers, in a preferred embodiment of chitosan and/or cellulose, modified with groups strongly binding copper ions, selected from the group containing ($-CH_2$-[8-hydroxyquinoline], $-CH_2$-[8-hydroxyquinoline-5-sulfonic acid], 2-pyridylmethyl).

Further, the invention includes the polymer for use as a medicament, specifically as an orally administered preparation for preventing and mitigating the consequences of the Wilson's disease. The polymer passes through the gastrointestinal tract with food and captures and binds copper released from the diet during digestion. Polymeric sorbent

at the same time captures and binds also the copper secreted into the digestive tract, thus further shifting the balance of copper in the organism towards excretion. As the polymers according to the invention are in general non-absorbable from the digestive tract, they are completely non-toxic and they are completely eliminated through the stools.

The polymeric matrices used are particularly biologically very well tolerated, however in a human digestive tract non-biodegradable, biopolymers (cellulose and chitosan), modified by the chelating groups. The covalently bound chelating group is capable of binding copper(II) ions in the environment of the stomach content (pH 2 to 4), and copper is not released from the sorbent in the intestinal contents (pH in average of 6.8) even in the presence of amino-acids released from the diet in relevant amount (especially amino acids strongly complexing copper ions, such as L-cysteine and L-histidine), and zinc(II) ions as the main competing factors.

The subject of the present invention is also a method of preparation of the above described functionalized polymers, wherein the polymer, carrying $NH_2$ or $NHCOCH_3$ groups as substituents $R_3$, is first subjected to a reaction with $C_2$ to $C_8$ dialdehyde or with epichlorohydrin or with epibromohydrin, which causes cross-linking, and then to Mannich reaction with formaldehyde and 8-hydroxyquinoline or 8-hydroxyquinoline-5-sulfonic acid.

The invention also provides a method of preparation of the above described functionalized polymers, wherein the polymer carrying H as substituents $R_1$ and $R_2$, and possibly also OH as substituent $R_3$, is first subjected to a reaction with allylhalogenide, the product of which is further subjected to reaction with peracetic acid, and the resulting epoxide ring reacts with a reagent selected from 2-aminoethanol, 1-amino-2-propanol, 2-amino-1-propanol, ammonia, ($C_1$-$C_6$)alkylamine, hydroxy($C_1$-$C_6$)alkylamine, (2-pyridylmethyl)amine, bis(2-pyridylmethyl)amine, and the resulting product then eventually reacts in Mannich reaction with formaldehyde and 8-hydroxyquinoline or 8-hydroxyquinoline-5-sulfonic acid.

<u>Brief description of figures</u>

Figure 1 shows FT-IR spectrum of particles of cross-linked chitosan according to Example 1.

Figure 2 shows comparison of $^{13}C$ CP/MAS NMR spectra of particles of cross-linked chitosan (dashed line) and particles containing bound 8-hydroxyquinoline (full line) according to Example 1.

Figure 3 shows FT-IR spectrum of particles of chitosan according to Example 1 containing bound 8-hydroxyquinoline.

Figure 4 shows the distribution of size of particles obtained from Mie light scattering on particles of chitosan according to Example 1 containing bound 8-hydroxyquinoline.

Figure 5 Radioactivity of internal organs of experimental animals in percentage of administered activity (corrected to decay from the time of administration) after administration of the particles according to Example 1 after 8 h (a) and after 24 h (b) versus reference animals after 8 h (c) and after 24 h (d).

Figure 6 shows FT-IR spectrum of allylated cellulose (full line) and glycidyl cellulose (dashed line) according to Example 2.

Figure 7 shows FT-IR spectrum of *N*-methylamino cellulose according to Example 2.

Figure 8 shows FT-IR spectrum of particles of 8-hydroxyquinoline 8HQ-cellulose (full line) and glycidyl cellulose (dashed line) according to Example 2.

Figure 9 shows the distribution of size of particles obtained from Mie light scattering on particles according to Example 2 containing bound 8-hydroxyquinoline.

Figure 10: $^{13}C$ CP/MAS NMR spectrum of particles according to Example 2 containing 8-hydroxyquinoline bound on the modified cellulose.

Figure 11: Radioactivity of internal organs of experimental animals in percentage of administered activity (corrected to decay from the time of administration) after administration of the particles according to Example 2 after 8 h (a) and after 24 h (b).

<u>Examples</u>

The following examples are shown to further illustrate the invention, but they do not limit the claimed scope of the invention.

*Instruments and methods:*

Particles sizes were measured by Mie light scattering using the instrument Mastersizer 3000 laser particle size analyzer (Malvern Instruments Ltd., Great Britain) in water as a medium.

Infrared spectroscopy (FT-IR) was measured using the instrument PARAGON 1000 PC FT-IR spectrometer (Perkin Elmer Inc., Massachusetts, U.S.A.).

Solid state nuclear magnetic resonance spectra (ss NMR, type of experiment $^{13}C$ CP/MAS - "cross-polarization magic angle spinning") was measured using the 11.7 T NMR Spectrometer (Bruker Avance III HD 500 US/WB NMR Spectrometer, Bruker, Karlsruhe, Germany) in 3.2-mm $ZrO_2$ rotors at the frequency of rotation 20 kHz.

Elemental analysis was measured using the instrument Perkin Elmer 2400 Series II CHNS/O Elemental Analysis (Perkin Elmer Inc., Massachusetts, U.S.A.).

Radioactivity was measured using the ionization chamber Bqmetr 4 (Empos Ltd., Praha, Czech Republic).

Example 1

Chitosan (10 g, average molecular weight of about 40 000 g/mole, degree of deacetylation 75 to 85 %) was dissolved in 300 ml of 6% aqueous acetic acid. Next, 50 ml of sorbitan monooleate was mixed with 150 ml of paraffin oil. Then the solution of chitosan was added to the oil phase and the mixture was homogenized for 1 min using the homogenizer, during which 10 ml of 50% aqueous solution of glutaraldehyde was added. Then the mixture was stirred using an anchor stirrer for 30 min at 1 000 RPM. The suspension of particles of the cross-linked chitosan was then washed subsequently with chloroform, ethanol, and water (final washing 7 x 24 h), and stored under water. The average size of the particles was 50 micrometers (optical microscopy, at equilibrium imbibition by water). CHN: C 38.09 %, H 8.27 %, N 3.51 %. Infra-red spectroscopy (FT-IR) - refer to **Figure. 1,** solid state nuclear magnetic resonance ($^{13}C$-ssNMR) refer to **Figure 2.**

Cross-linked chitosan particles (3.00 g in dry matter, 7.52 mmole N) were reacted with 8-hydroxyquinoline (7.00 g, 48.7 mmole) and 40 % aqueous formaldehyde (8.95 ml, 200 mmole) in 50 ml methanol at room temperature for 7 days and occasional shaking. The polymer was then washed subsequently with methanol, aqueous hydrochloric acid (1 mole/l), water, aqueous ammonium acetate (0.5 mole/l), water, and dried. Yield 3 g. CHN C 57.50 %, H 5.84 %, N 6.86 %. FT-IR refer to **Figure. 3,** $^{13}C$-ssNMR refer to **Figure.** 2. The content of 8-hydroxyquinoline O8HQ (in mmole/g) was determined from the elemental analysis of carbon and $^{13}C$-ssNMR according to the formula:

$$\mathrm{O8HQ} = 1000 * (I_{8HQ} / I_{\Sigma}) * (\omega_C / \omega C_{8HQ}) * (1000 / M_{8HQ})\ \mathrm{mmole/g} = (I_{8HQ} / I_{\Sigma}) * \omega_C * 9.2506\ \mathrm{mmole/g}$$

wherein $I_{8HQ}$ is the integral signal of aromatic carbons of 8-hydroxyquinoline with chemical shifts of 114 - 156 ppm, $I_{\Sigma}$ is a sum of integral signals of all carbons in the sample, $\omega_C$ is the weight fraction of carbon in the sample, $\omega C_{8HQ}$ is the weight fraction of carbon in 8-hydroxyquinoline ($\omega C_{8HQ}$ = 0.7447) and $M_{8HQ}$ is a molecular weight of 8-hydroxyquinoline ($M_{8HQ}$ = 145.16), and the result was O8HQ = 2.24 mmole/g.

The size of particles according to the Mie light scattering D [4.3] = 68.0 micrometers with the values of $D_v(10)$ = 31.6 micrometers, $D_v(50)$ = 63.0 micrometers, $D_v(90)$ = 113 micrometers, for distributions refer to **Figure 4.**

Stock solution of radio-copper was prepared by dilution of 800 μl $^{64}CuCl_2$ in aqueous hydrochloric acid (0.05 mole/l HCl, 680 MBq $^{64}Cu$) in 9.2 ml of aqueous solution of sodium chloride (0.15 mole/l). Healthy female Wistar rats (11 weeks old, average weight 220 g) were divided into 4 groups of 6 animals. Two groups of animals were administered by gastric gavage into the stomach only radio-copper stock solution (250 μl per animal, reference) and two groups were administered radio-copper stock solution (250 μl per animal) together with particles according to Example 1 (50 mg of dry matter per animal). After 8 hours, one reference group, which was given only the solution of radio-copper, and one experimental group that received also the particles according to this example, were sacrificed, and an autopsy was performed and radioactivity of different organs was measured (stomach, large intestine, small intestine, appendix, blood, liver, kidney, lung, heart, spleen, the rest of the body). After 24 hours, the remaining animals were killed, and their autopsy was performed, and the radioactivity of the organs was measured as for the previous two groups. The values of radioactivity were recalculated to decay to the time of the beginning of the experiment using the value of the half-life of $^{64}Cu$ 12.7 h.

**Figure 5** shows a very strong inhibition of radio-copper absorption from the digestive tract (and its subsequent re-deposition in internal organs, especially in the liver) by the particles according to Example 1 in comparison with the reference group both after 8 h (a) and after 24 h from the administration (b) in comparison with the reference after 8 h (c) and 24 h (d).

Example 2

Microcrystalline cellulose (16.67 g) was dissolved in a solution of lithium chloride (30 g, 0.71 mole) in dimethylacetamide (500 ml) by heating to 80 °C for the period of 7 days with stirring. Then the solution was cooled down to 0 °C, sodium hydroxide was added (123.4 g, 3.09 mole), and then allylbromide by drops with stirring (262 ml, 3.03 mole).

The mixture was then stirred and heated to 80 °C for 5 hours and then stirred for additional 12 hours at room temperature. Water was then added until dissolution, and the aqueous phase was extracted twice with chloroform. The chloroform layers were combined, evaporated and the residue was azeotropically final-dried with toluene. The yield of allylated cellulose as yellowish oil was 6 g. Elemental analysis C 58.42 %, H 7.46 %. FT-IR refer to **Figure 6.**

Thirty-five percent aqueous peracetic acid (107.5 g, 0.495 mole) was dropped into a mixture of allylated cellulose (5 g), sodium acetate (0.076 mole) and dichloromethane (400 ml). The reaction mixture was stirred at room temperature for 16 h, then diluted with water, the dichloromethane layer was separated, shaken out with 10% aqueous sodium bicarbonate and then with water, dried with anhydrous magnesium sulfate and evaporated. The yield of glycidyl cellulose was 3.5 g. Elemental analysis: C 51.92 %, H 6.19 %. FT-IR refer to **Figure 6.**

Glycidyl cellulose (3 g) was stirred and reacted with 40% aqueous methylamine (70 ml, 0.462 mole) for 72 h at room temperature. The resulting polymer was washed with water and methanol and dried. The yield of *N*-methylamino cellulose was 2.72 g. Elemental analysis C 48.09 %, H 9.44 %, N 6.65 %. FT-IR refer to **Figure 7.**

*N*-Methylamino cellulose (1.39 g), 40% aqueous formaldehyde (2.37 ml, 64 mmole), 8-hydroxyquinoline (1.80 g, 12.4 mmole) and methanol (7.9 ml) reacted at room temperature for 10 days with occasional stirring. The product was then washed with water and methanol using a funnel, dried and milled using ball mill. The yield was 2.47 g. Elemental analysis C 62.97 %, H 6.86 %, N 8.34 %. FT-IR refer to **Figure 8.**

The particle size was according to the Mie light scattering D [4.3] = 71.8 micrometers with the values of $D_V(10)$ = 13.2 micrometers, $D_V(50)$ = 56.6 micrometers, $D_V(90)$ = 152 micrometers, for distribution refer to **Figure 9.**

The content of 8-hydroxyquinoline was calculated from $^{13}$C- ssNMR (for the spectrum refer to **Figure 10)** as shown in the Example 1. The resulting O8HQ = 3.72 mmole/g.

The polymer according to Example 2 was tested for inhibition of intake of radio-copper in the same manner as described in Example 1. **Figure 11** shows very strong inhibition of absorption of radio-copper from the digestive tract (and subsequently its re-deposition in the internal organs, especially the liver) by the particles according to Example 2 in comparison with the reference groups, both after 8 h (a) and after 24 h from the administration (b), in comparison with the reference (refer to **Figure 5** (c) and (d), the reference is the same as in the Example 1).

Example 3

Micro-particles were prepared according to Example 1, wherein 8-hydroxyquinoline-5-sulfonic acid synthesis in equimolar amount (10.97 g, 48.7 mmole) was used instead of 8-hydroxyquinoline in the last step. The yield was 3.5 g, content of sulfur 4.65 weight %. The content of the ligand O8HQS (in mmole/g) was calculated from the elemental analysis of the content of sulfur according to the formula:

$$\mathrm{O8HQS} = \omega_S / 3.206$$

wherein $\omega_S$ is the content of sulfur in weight %. The resulting content O8HQS = 1.45 mmole/g.

Example 4

Micro-particles were prepared according to the Example 2, wherein the last step was omitted and in the last but one step the aqueous methylamine was replaced with bis(2-pyridylmethyl)amine (4.5 g, 22.6 mmole). The reaction was performed in 2-propyl alcohol (10 ml) at 75 °C for 16 h. The yield was 2.85 g, nitrogen content 11.32 weight %. The content of the bis(2-pyridylmethyl)amine ligand (ODPA, in mmole/g) was calculated from the elemental analysis of the content of nitrogen according to the formula:

$$\mathrm{ODPA} = \omega_N / 4{,}2$$

where $\omega_N$ is the content of nitrogen in weight %. The resulting content ODPA = 2.70 mmole/g.

## Claims

**1.** A polysaccharide based polymer containing monomeric units of a general formula I

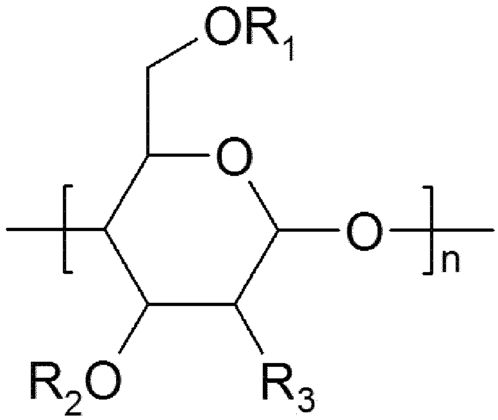

(I)

wherein

$R_1$ is selected from a group containing H, $-CH_2-CH(OH)-CH_2-NR_4R_5$;
$R_2$ is selected from a group containing H, $-CH_2-CH(OH)-CH_2-NR_4R_5$;
$R_3$ is selected from a group containing OH, $-O-CH_2-CH(OH)-CH_2-NR_4R_5$, $NR_4R_5$;
n is an integer in the range of from 60 to 50 000;
$R_4$ is selected from a group containing H, $(C_1-C_6)$alkyl, hydroxy$(C_1-C_6)$alkyl, $-CH_2$-[8-hydroxyquinoline], $-CH_2$-[8-hydroxyquinoline-5-sulfonic acid], 2-pyridylmethyl;
if $R_3$ is $NR_4R_5$, then from 0 to 50 mol. % of substituents $R_4$ in this group may be acetyl;
$R_5$ is selected from a group containing H, $(C_1-C_6)$alkyl, hydroxy$(C_1-C_6)$alkyl, $-CH_2$-[8-hydroxyquinoline], $-CH_2$-[8-hydroxyquinoline-5-sulfonic acid], 2-pyridylmethyl;
wherein at least 5 mol. % of the total amount of substituents $R_1$, $R_2$, and $R_3$ in the polymer carry the substituent $NR_4R_5$, wherein $R_4$ and/or $R_5$ is a group selected from the group containing $-CH_2$-[8-hydroxyquinoline], $-CH_2$-[8-hydroxyquinoline-5-sulfonic acid], 2-pyridylmethyl;
and wherein the polymer is cross-linked so that at least 0.1 mol. % of monomeric units are covalently bridged.

2. The polymer according to claim 1, **characterized in that** covalent bridging of monomeric units is done through the following bridges:

- in case that $R_3$ is $NR_4R_5$, then substituents $R_4$ and/or $R_5$ present in this group of two monomeric units form together the bridge $=C-(CH_2)_n-C=$, where n is from 0 to 8, or $-CH_2-CH(OH)-CH_2-$;
- in case that $R_3$ is not $NR_4R_5$, then $R_3 = OR_3'$, and one or more of the substituents $R_1$, $R_2$, $R_3'$ of one monomeric unit form(s) together with $R_1$, $R_2$ or $R_3'$ of another unit a bridge with the structure of $-CH_2-CH(OH)-CH_2-N(R_6)-CH_2-CH(OH)-CH_2-$, where $R_6$ is selected from a group containing H, $(C_1-C_6)$alkyl, hydroxy$(C_1-C_6)$alkyl, $-CH_2$-[8-hydroxyquinoline],$-CH_2$-[8-hydroxyquinoline-5-sulfonic acid], 2-pyridylmethyl.

3. The polymer according to claim 1 or 2, **characterized in that** at least 1 mol. % and maximum 50 mol. % of monomeric units are covalently bridged, preferably from 3 to 10 mol. % of monomeric units are covalently bridged.

4. The polymer according to any one of the preceding claims, **characterized in that** the monomeric units are monomeric units of cellulose, functionalized by the substituents $R_1$, $R_2$, $R_3$, as defined above, and $R_3$ is not $NR_4R_5$, or the monomeric units are monomeric units of chitosan, functionalized with the substituents $R_1$, $R_2$, $R_3$, as defined above, and $R_3$ is $NR_4R_5$.

5. The polymer according to any one of the preceding claims, **characterized in that** it is in a form of microparticles of sizes from 1 to 1 000 micrometers.

6. The polymer according to any one of the preceding claims for use as a medicament.

7. The polymer according to any one of the claims 1 to 5 for use as an orally administered preparation for prevention and/or treatment of Wilson's disease.

8. A method of preparation of the polysaccharide based polymer according to any one of the preceding claims, wherein the polymer, carrying $NH_2$ or $NHCOCH_3$ groups as substituents $R_3$, first undergoes a reaction with $C_2$ to $C_8$ dialdehyde or with epichlorohydrin or with epibromohydrin, which results in its cross-linking, and then to Mannich reaction with formaldehyde and 8-hydroxyquinoline or 8-hydroxyquinoline-5-sulfonic acid.

9. A method of preparation of the polysaccharide based polymer according to any one of the claims 1 to 7, wherein wherein the polymer carrying H as substituents $R_1$ and $R_2$, and possibly also OH as substituent $R_3$, first undergoes a reaction with allylhalogenide, the product of which further undergoes a reaction with peracetic acid, and the resulting epoxide ring reacts with a reagent selected from 2-aminoethanol, 1-amino-2-propanol, 2-amino-1-propanol, ammonia, $(C_1\text{-}C_6)$alkylamine, hydroxy$(C_1\text{-}C_6)$alkylamine, (2-pyridylmethyl)amine, bis(2-pyridylmethyl)amine, and the resulting product then eventually reacts in Mannich reaction with formaldehyde and 8-hydroxyquinoline or 8-hydroxyquinoline-5-sulfonic acid.

**Patentansprüche**

1. Ein Polymer auf Polysaccharidbasis, das Monomereinheiten einer allgemeinen Formel I enthält

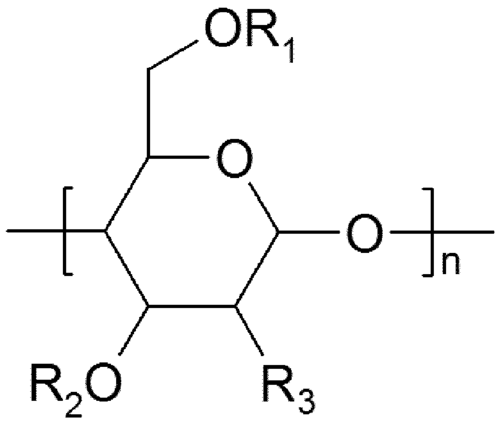

(I)

worin

$R_1$ ausgewählt ist aus einer Gruppe, die H, $-CH_2\text{-}CH(OH)\text{-}CH_2\text{-}NR_4R_5$ enthält;
$R_2$ ausgewählt ist aus einer Gruppe, die H, $-CH_2\text{-}CH(OH)\text{-}CH_2\text{-}NR_4R_5$ enthält;
$R_3$ ausgewählt ist aus einer Gruppe, die OH, $-O\text{-}CH_2\text{-}CH(OH)\text{-}CH_2\text{-}NR_4R_5$, $NR_4R_5$ enthält;
n ist eine ganze Zahl im Bereich von 60 bis 50 000;
$R_4$ ist ausgewählt aus einer Gruppe, die H, $(C_1\text{-}C_6)$-Alkyl, Hydroxy$(C_1\text{-}C_6)$alkyl, $-CH_2$-[8-Hydroxychinolin], $-CH_2$-[8-Hydroxychinolin-5-sulfonsäure], 2-Pyridylmethyl enthält;
wenn $R_3$ $NR_4R_5$ ist, dann von 0 bis 50 mol. % der Substituenten $R_4$ in dieser Gruppe können Acetyl sein;
$R_5$ ausgewählt ist aus einer Gruppe, die H, $(C_1\text{-}C_6)$-Alkyl, Hydroxy$(C_1\text{-}C_6)$alkyl, $-CH_2$-[8-Hydroxychinolin], $-CH_2$-[8-Hydroxychinolin-5-sulfonsäure], 2-Pyridylmethyl enthält;
wobei mindestens 5 mol. % der Gesamtmenge der Substituenten $R_1$, $R_2$ und $R_3$ im Polymer tragen den Substituenten $NR_4R_5$, worin $R_4$ und/oder $R_5$ ausgewählt ist aus der Gruppe, die-$CH_2$-[8-Hydroxychinolin], $-CH_2$-[8-Hydroxychinolin-5-sulfonsäure], 2-Pyridylmethyl enthält;
und wobei das Polymer so vernetzt ist, dass mindestens 0,1 mol. % der Monomereinheiten sind kovalent verbrückt.

2. Polymer nach Anspruch 1, **dadurch gekennzeichnet, dass** die kovalente Verbrückung von Monomereinheiten

über folgende Brücken erfolgt:

- falls $R_3$ $NR_4R_5$ ist, bilden die Substituenten $R_4$ und/oder $R_5$ von zwei Monomereinheiten zusammen die Brücke $=C-(CH_2)_nC=$, worin n 0 bis 8 ist, oder $-CH_2-CH(OH)-CH_2-$;
- falls $R_3$ nicht $NR_4R_5$ ist, dann ist $R_3 = OR_3'$ und einer oder mehrere der Substituenten $R_1$, $R_2$, $R_3'$ einer monomeren Einheit bilden zusammen mit $R_1$, $R_2$ oder $R_3'$ einer anderen Einheit eine Brücke mit die Struktur $-CH_2-CH(OH)-CH_2-N(R_6)-CH_2-CH(OH)-CH_2-$, wobei $R_6$ aus einer Gruppe ausgewählt ist, die H, ($C_1$-$C_6$)-Alkyl, Hydroxy($C_1$-$C_6$)Alkyl, $-CH_2$-[8-Hydroxychinolin], $-CH_2$-[8-Hydroxychinolin-5-sulfonsäure], 2-Pyridylmethyl enthält.

**3.** Polymer nach Anspruch 1 oder 2, **dadurch gekennzeichnet, dass** mindestens 1 Mol. % und maximal 50 mol. % der Monomereinheiten sind kovalent verbrückt, vorzugsweise 3 bis 10 Mol. % der Monomereinheiten sind kovalent verbrückt.

**4.** Das Polymer nach einem der vorhergehenden Ansprüche, **dadurch gekennzeichnet, dass** die Monomereinheiten sind Monomereinheiten von Cellulose, die durch die Substituenten $R_1$, $R_2$, $R_3$ wie oben definiert funktionalisiert sind und $R_3$ nicht $NR_4R_5$ ist, oder die Monomereinheiten sind Monomereinheiten von Chitosan, funktionalisiert mit den Substituenten $R_1$, $R_2$, $R_3$, wie oben definiert, und $R_3$ ist $NR_4R_5$.

**5.** Das Polymer nach einem der vorhergehenden Ansprüche, **dadurch gekennzeichnet, dass** es in Form von Mikropartikeln mit Größen von 1 bis 1000 Mikrometern ist.

**6.** Das Polymer nach einem der vorhergehenden Ansprüche zur Verwendung als Medikament.

**7.** Polymer nach einem der Ansprüche 1 bis 5 zur Verwendung als oral verabreichtes Präparat zur Vorbeugung und/oder Behandlung von Morbus Wilson.

**8.** Verfahren zur Herstellung des Polymers auf Polysaccharidbasis nach einem der vorhergehenden Ansprüche, wobei das Polymer, das $NH_2$- oder $NHCOCH_3$-Gruppen als Substituenten $R_3$ trägt, zuerst eine Reaktion mit $C_2$ bis $C_8$-Dialdehyd oder mit Epichlorhydrin oder mit Epibromhydrin eingeht, was führt zu seine Vernetzung, und dann eine Mannich-Reaktion mit Formaldehyd und 8-Hydroxychinolin oder 8-Hydroxychinolin-5-sulfonsäure eingeht.

**9.** Verfahren zur Herstellung des Polymers auf Polysaccharidbasis nach einem der Ansprüche 1 bis 7, wobei das Polymer, das H als Substituenten $R_1$ und $R_2$ trägt, und möglicherweise auch OH als Substituenten $R_3$ trägt, zuerst eine Reaktion mit Allylhalogenid eingeht, und das Produkt davon weiter eine Reaktion mit Peressigsäure eingeht, und der resultierende Epoxidring reagiert mit einem Reagenz, ausgewählt aus 2-Aminoethanol, 1-Amino-2-propanol, 2-Amino-1-propanol, Ammoniak, ($C_1$-$C_6$)-Alkylamin, Hydroxy($C_1$-$C_6$)-Alkylamin, (2-Pyridylmethyl)amin, Bis(2-pyridylmethyl)amin und das resultierende Produkt reagiert dann schließlich in der Mannich-Reaktion mit Formaldehyd und 8-Hydroxychinolin oder 8-Hydroxychinolin-5-sulfonsäure.

## Revendications

**1.** Un polymère à base de polysaccharide contenant des unités monomères de formule générale I

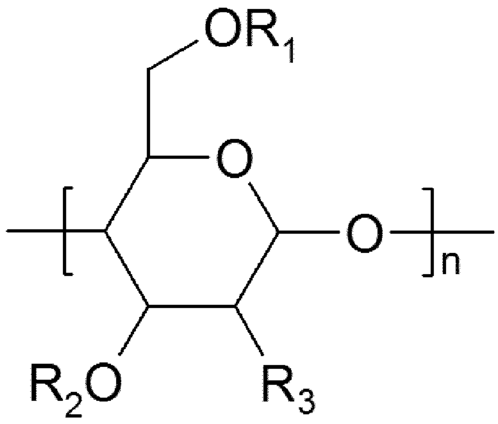

(I)

où

$R_1$ est sélectionné dans un groupe contenant H, $-CH_2-CH(OH)-CH_2-NR_4R_5$;
$R_2$ est sélectionné dans un groupe contenant H, $-CH_2-CH(OH)-CH_2-NR_4R_5$;
$R_3$ est sélectionné dans un groupe contenant OH, $-O-CH_2-CH(OH)-CH_2-NR_4R_5$, $NR_4R_5$;
n est un entier dans la rangée de 60 à 50 000;
$R_4$ est sélectionné dans un groupe contenant H, $(C_1-C_6)$alkyle, hydroxy$(C_1-C_6)$alkyle, $-CH_2$-[8-hydroxyquinoléine], $-CH_2$-[8-hydroxyquinoléine-5-sulfonique acide], 2-pyridylméthyle; si $R_3$ est $NR_4R_5$, alors de 0 à 50 mol. % des substituants $R_4$ dans ce groupe peuvent être acétyle;
$R_5$ est sélectionné dans un groupe contenant H, $(C_1-C_6)$alkyle, hydroxy$(C_1-C_6)$alkyle, $-CH_2$-[8-hydroxyquinoléine], -CH2-[8-hydroxyquinoline-5-sulfonique acide], 2-pyridylméthyle;
où au moins 5 mol. % de la quantité totale de substituants $R_1$, $R_2$ et $R_3$ dans le polymère portent le substituant $NR_4R_5$, où $R_4$ et/ou $R_5$ est un groupe sélectionné dans le groupe contenant $-CH_2$-[8-hydroxyquinoléine], $-CH_2$-[8-hydroxyquinoléine-5-sulfonique acide], 2-pyridylméthyle;
et où le polymère est réticulé de sorte qu'au moins 0,1 mol. % des unités monomères sont pontées de liaison covalente.

2. Le polymère selon la revendication 1, **caractérisé en ce que** le pont covalent des unités monomères se fait via les ponts suivants:

- dans le cas où $R_3$ est $NR_4R_5$, alors les substituants $R_4$ et/ou $R_5$ présents dans ce groupe de deux unités monomères forment ensemble le pont $=C-(CH_2)_nC=$, où n est de 0 à 8, ou $-CH_2-CH(OH)-CH_2-$;
- dans le cas où $R_3$ n'est pas $NR_4R_5$, alors $R_3 = OR_3'$, et un ou plusieurs des substituants $R_1$, $R_2$, $R_3$ d'une unité monomère forme(s) un pont avec $R_1$, $R_2$ ou $R_3'$ d'une autre unité monomère, le pont ayant la structure de $-CH_2-CH(OH)-CH_2-N(R_6)-CH_2-CH(OH)-CH_2-$, où $R_6$ est sélectionné dans un groupe contenant H, $(C_1-C_6)$alkyle, hydroxy$(C_1-C_6)$alkyle,$-CH_2$-[8-hydroxyquinoléine], $-CH_2$-[8-hydroxyquinoléine-5-sulfonique acide], 2-pyridylméthyle.

3. Le polymère selon la revendication 1 ou 2, **caractérisé en ce qu'**au moins 1 mol. % et au maximum 50 mol. % des unités monomères sont pontées de liaisone covalente, de préférence de 3 à 10 mol. % des unités monomères sont pontées de liaisone covalente.

4. Le polymère selon l'une quelconque des revendications précédentes, **caractérisé en ce que** les unités monomères sont des unités monomères de cellulose, fonctionnalisées avec les substituants $R_1$, $R_2$, $R_3$, tels que définis ci-dessus, et $R_3$ n'est pas $NR_4R_5$, ou les unités monomères sont des unités monomères de chitosane, fonctionnalisées avec les substituants $R_1$, $R_2$, $R_3$, tels que définis ci-dessus, et $R_3$ est $NR_4R_5$.

5. Le polymère selon l'une quelconque des revendications précédentes, **caractérisé en ce qu'**il se présente sous la forme de microparticules de tailles de 1 à 1 000 micromètres.

**6.** Le polymère selon l'une quelconque des revendications précédentes pour utilisation comme médicament.

**7.** Le polymère selon l'une quelconque des revendications 1 à 5 pour utilisation comme préparation administrée par voie orale dans la prévention ou le traitement de la maladie de Wilson.

**8.** Un procédé de préparation du polymère à base de polysaccharide selon l'une quelconque des revendications précédentes, dans lequel le polymère, portant des groupes $NH_2$ ou $NHCOCH_3$ en tant que substituants $R_3$, subit d'abord une réaction avec le ($C_2$ à $C_8$)dialdéhyde ou avec l'épichlorhydrine ou avec l'épibromohydrine, ce qui se traduit par sa réticulation, puis à la réaction de Mannich avec le formaldéhyde et la 8-hydroxyquinoléine ou l'acide 8-hydroxyquinoléine-5-sulfonique.

**9.** Le procédé de préparation du polymère à base de polysaccharide selon l'une quelconque des revendications 1 à 7, dans lequel le polymère portant H comme substituants $R_1$ et $R_2$, et éventuellement également OH comme substituant $R_3$, subit d'abord une réaction avec l'allylhalogénure, le produit de qui subit en outre une réaction avec l'acide peracétique, et le cycle époxyde résultant réagit avec un réactif choisi parmi le 2-aminoéthanol, le 1-amino-2-propanol, le 2-amino-1-propanol, l'ammoniac, le ($C_1$-$C_6$)alkylamine, l'hydroxy($C_1$-$C_6$) alkylamine, (2-pyridylméthyl)amine, bis(2-pyridylméthyl)amine, et le produit résultant réagit finalement dans la réaction de Mannich avec le formaldéhyde et la 8-hydroxyquinoléine ou l'acide 8-hydroxyquinoléine-5-sulfonique.

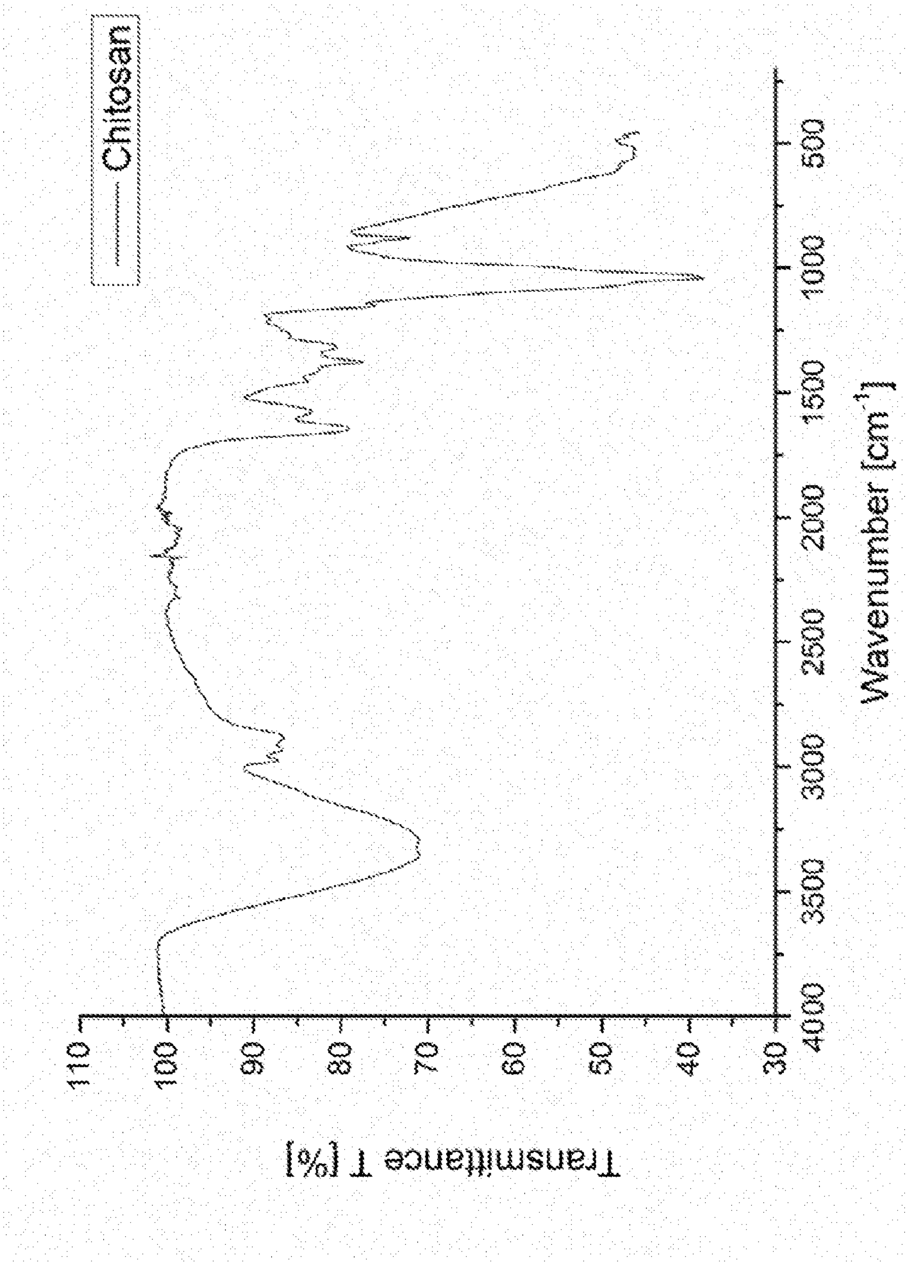
Chitosan
Transmittance T [%]
Wavenumber [cm-1]
110
100
90
80
70
60
50
40
30
4000
3500
3000
2500
2000
1500
1000
500

Figure 1

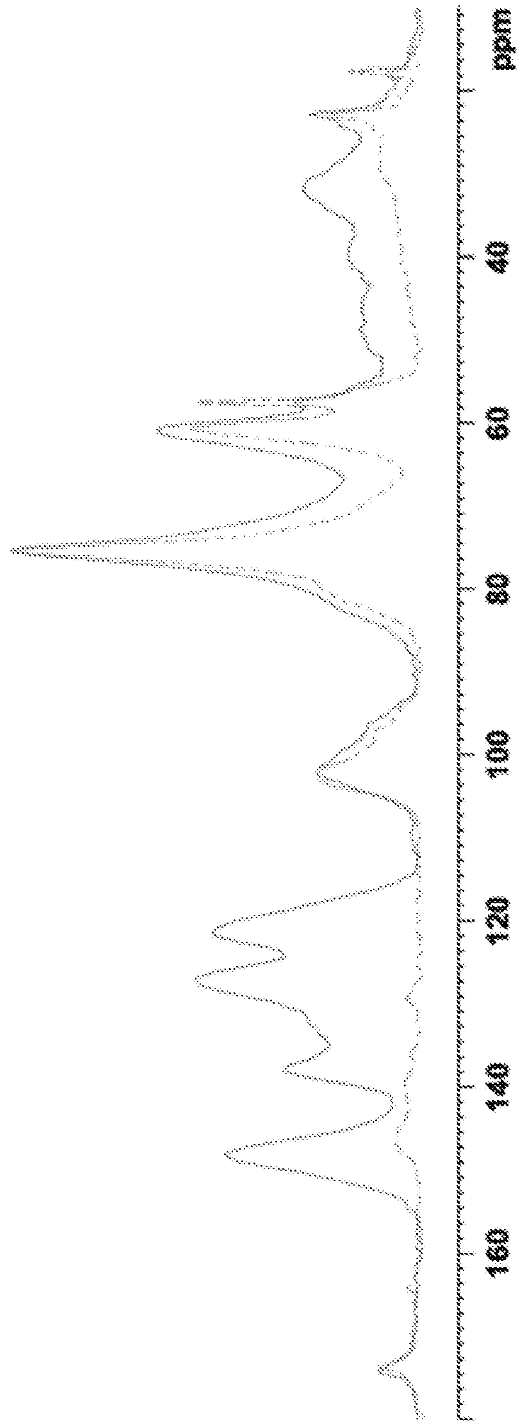


Figure 2

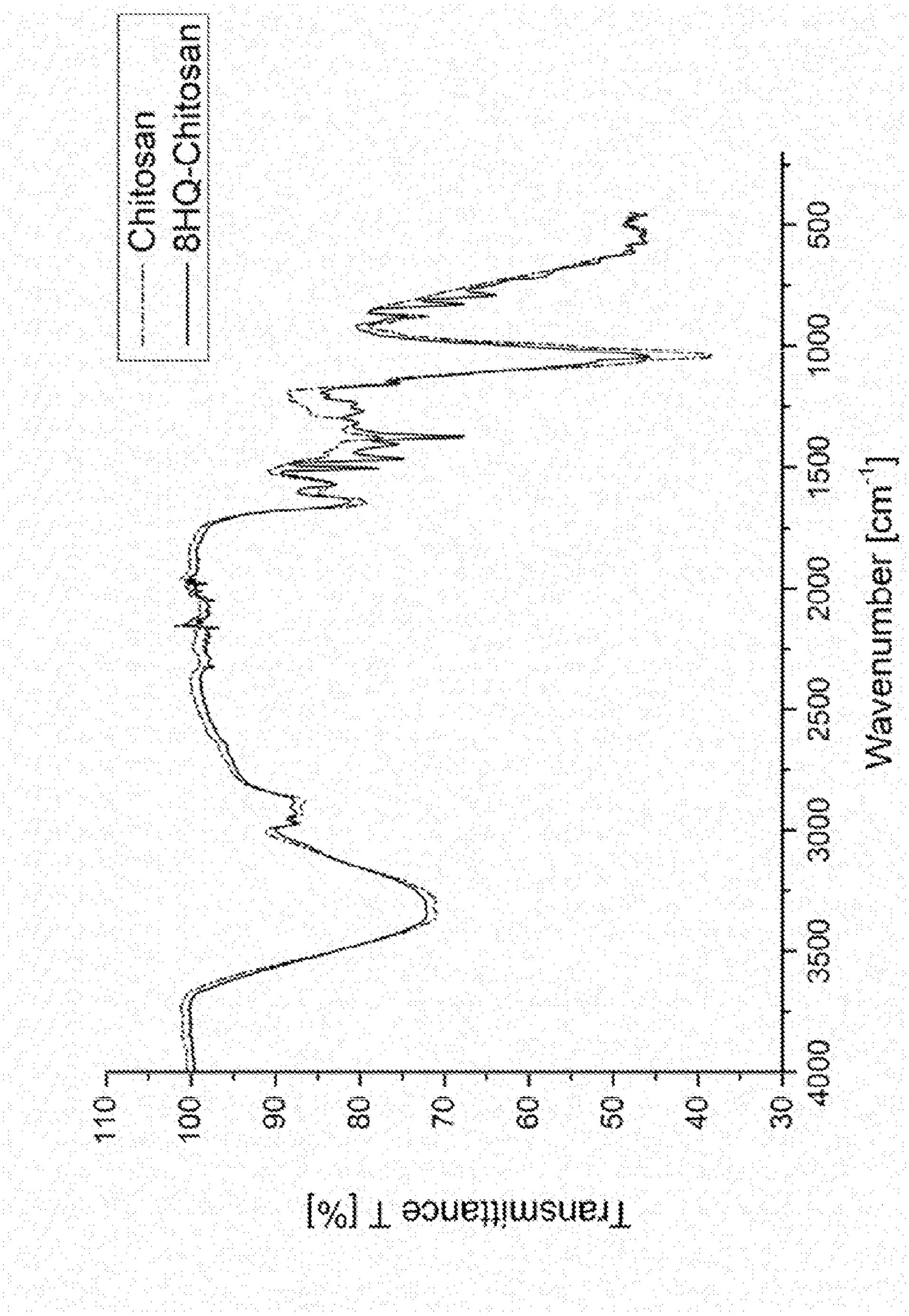
Chitosan
8HQ-Chitosan
Transmittance T [%]
Wavenumber [cm-1]
110
100
90
80
70
60
50
40
30
4000
3500
3000
2500
2000
1500
1000
500

Figure 3

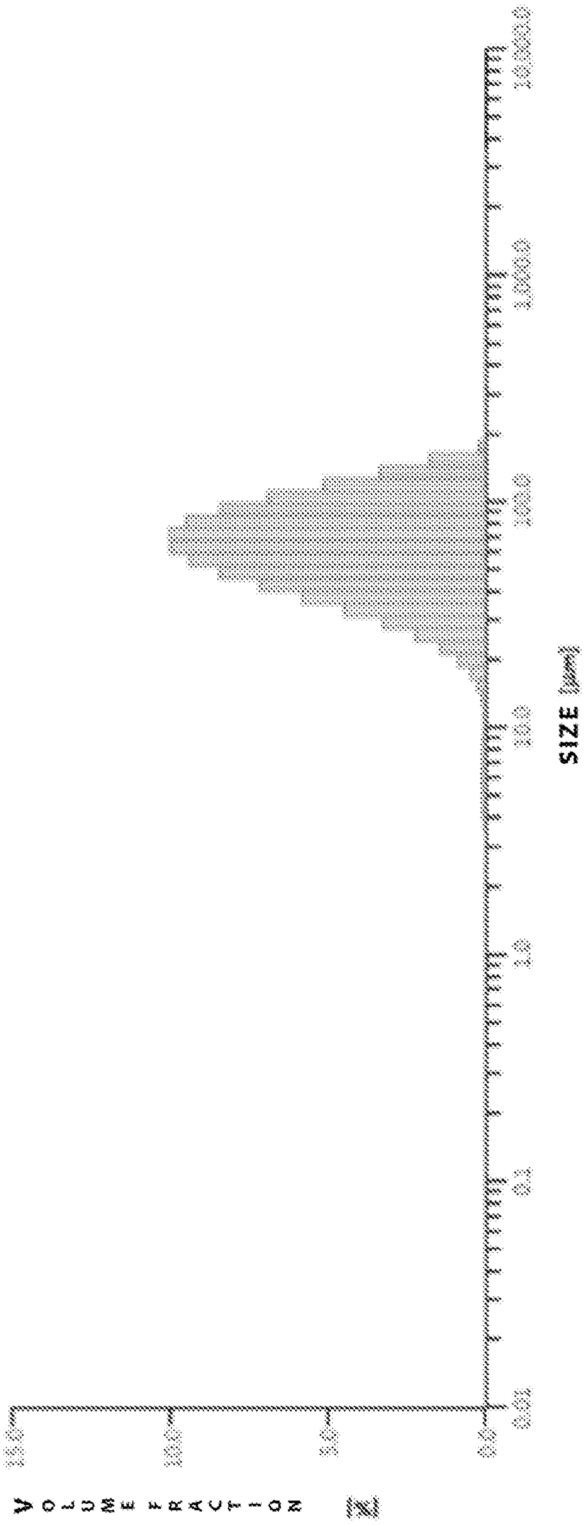
VOLUME FRACTION [%]
0.0
5.0
10.0
15.0
SIZE [μm]
0.01
0.1
1.0
10.0
100.0
1000.0
10000.0

Figure 4

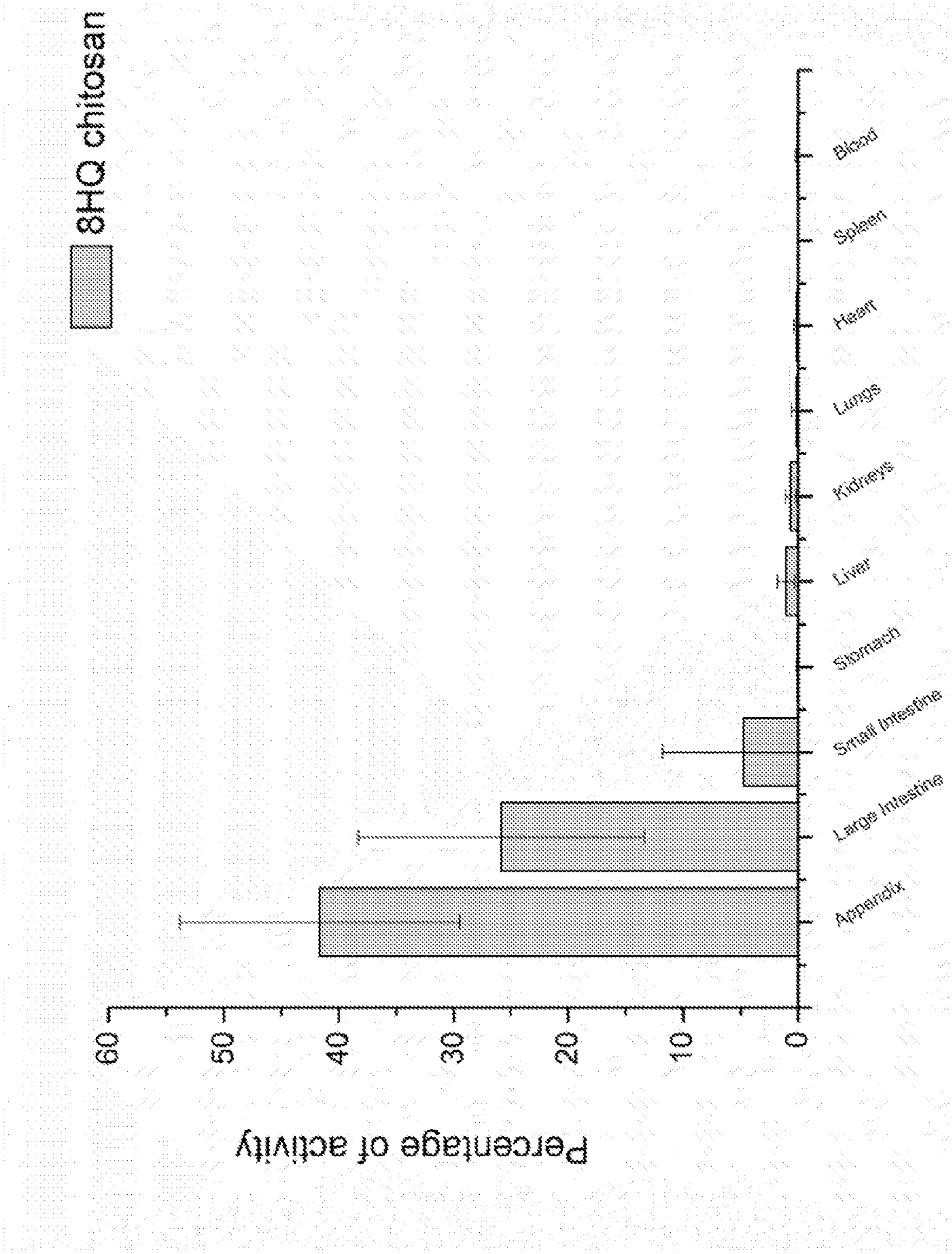

8HQ chitosan
Percentage of activity
0
10
20
30
40
50
60
Appendix
Large Intestine
Small Intestine
Stomach
Liver
Kidneys
Lungs
Heart
Spleen
Blood


Figure 5a

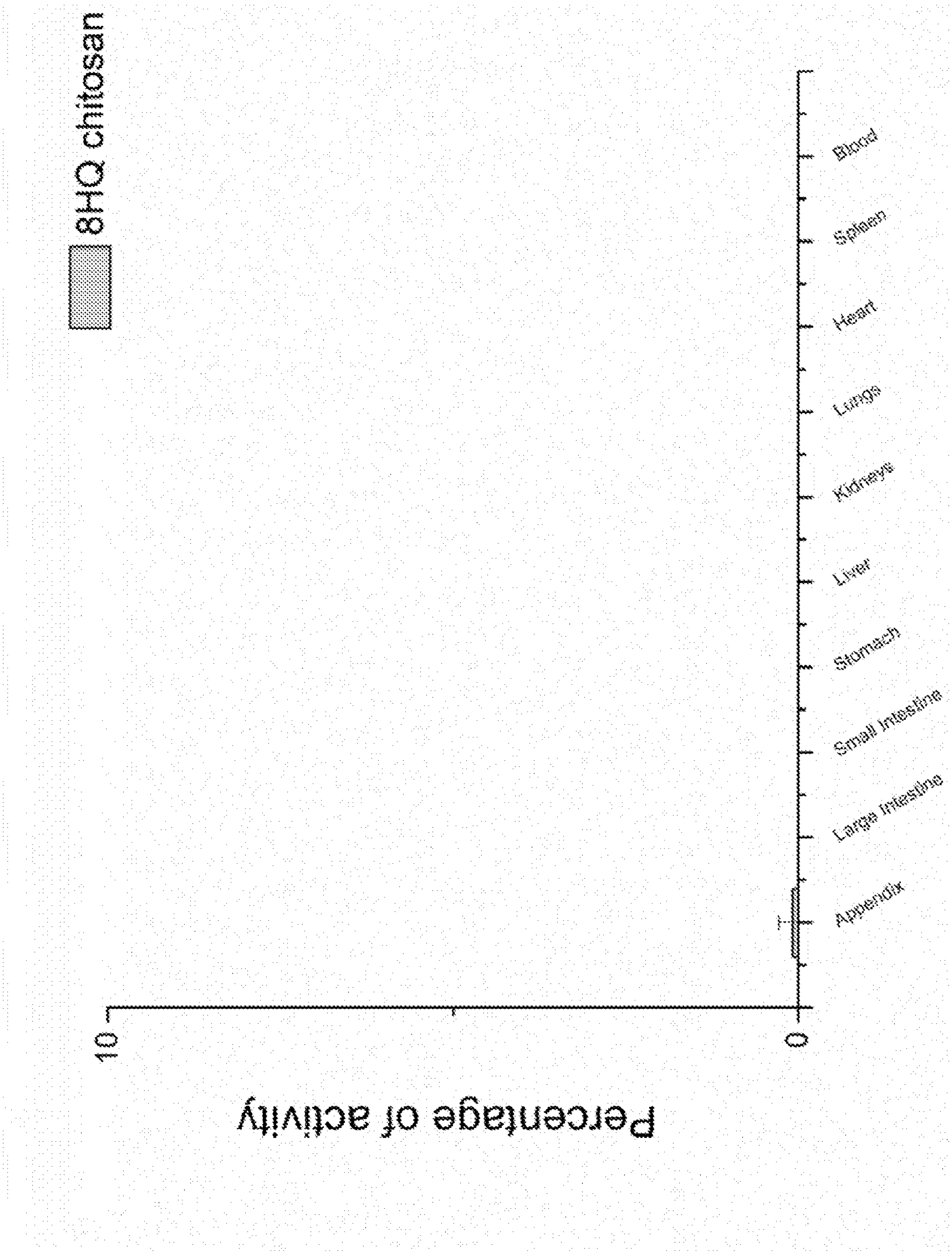
8HQ chitosan
Percentage of activity
10
0
Blood
Spleen
Heart
Lungs
Kidneys
Liver
Stomach
Small Intestine
Large Intestine
Appendix

Figure 5b

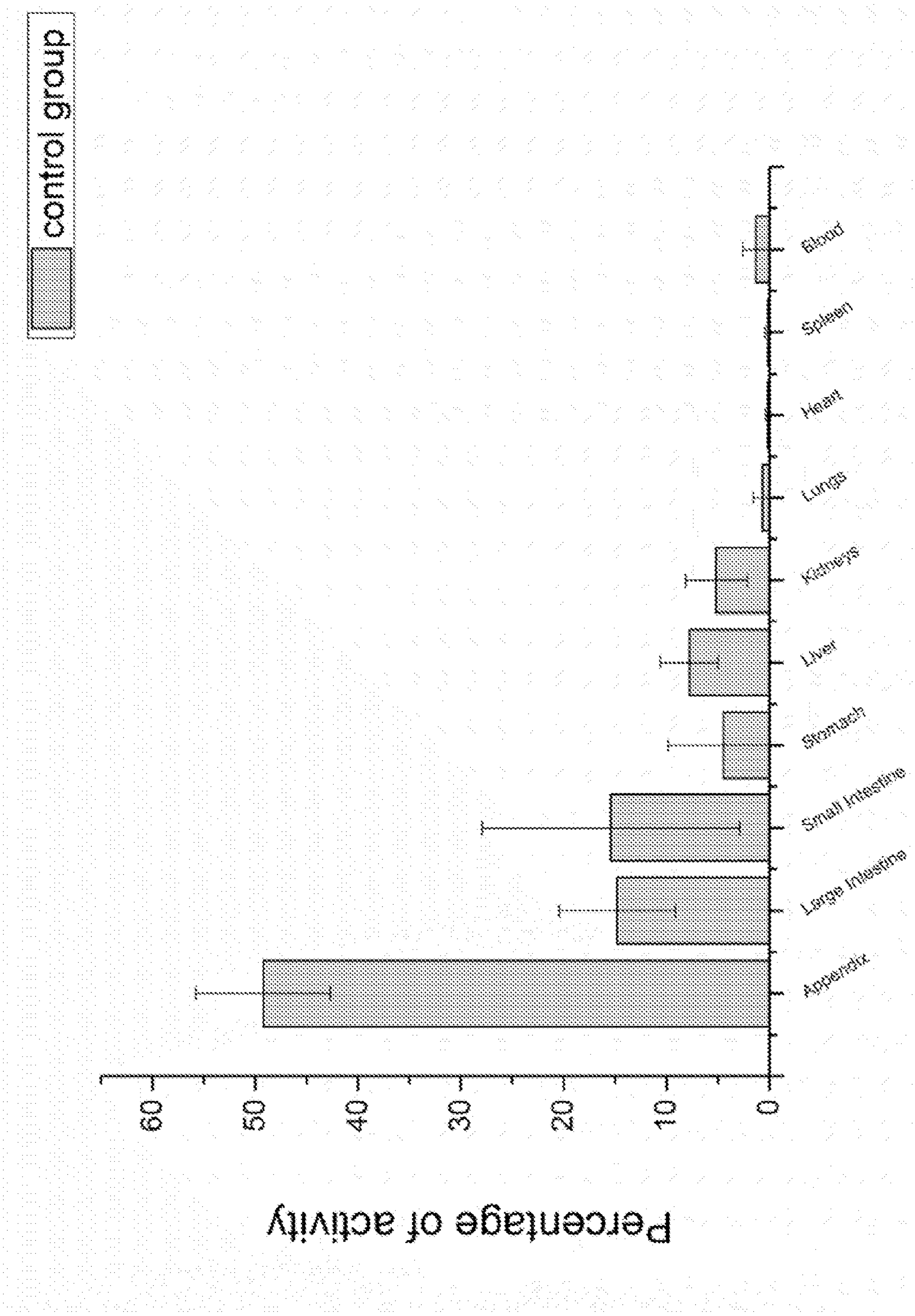
control group
Percentage of activity
0
10
20
30
40
50
60
Appendix
Large Intestine
Small Intestine
Stomach
Liver
Kidneys
Lungs
Heart
Spleen
Blood

Figure 5c

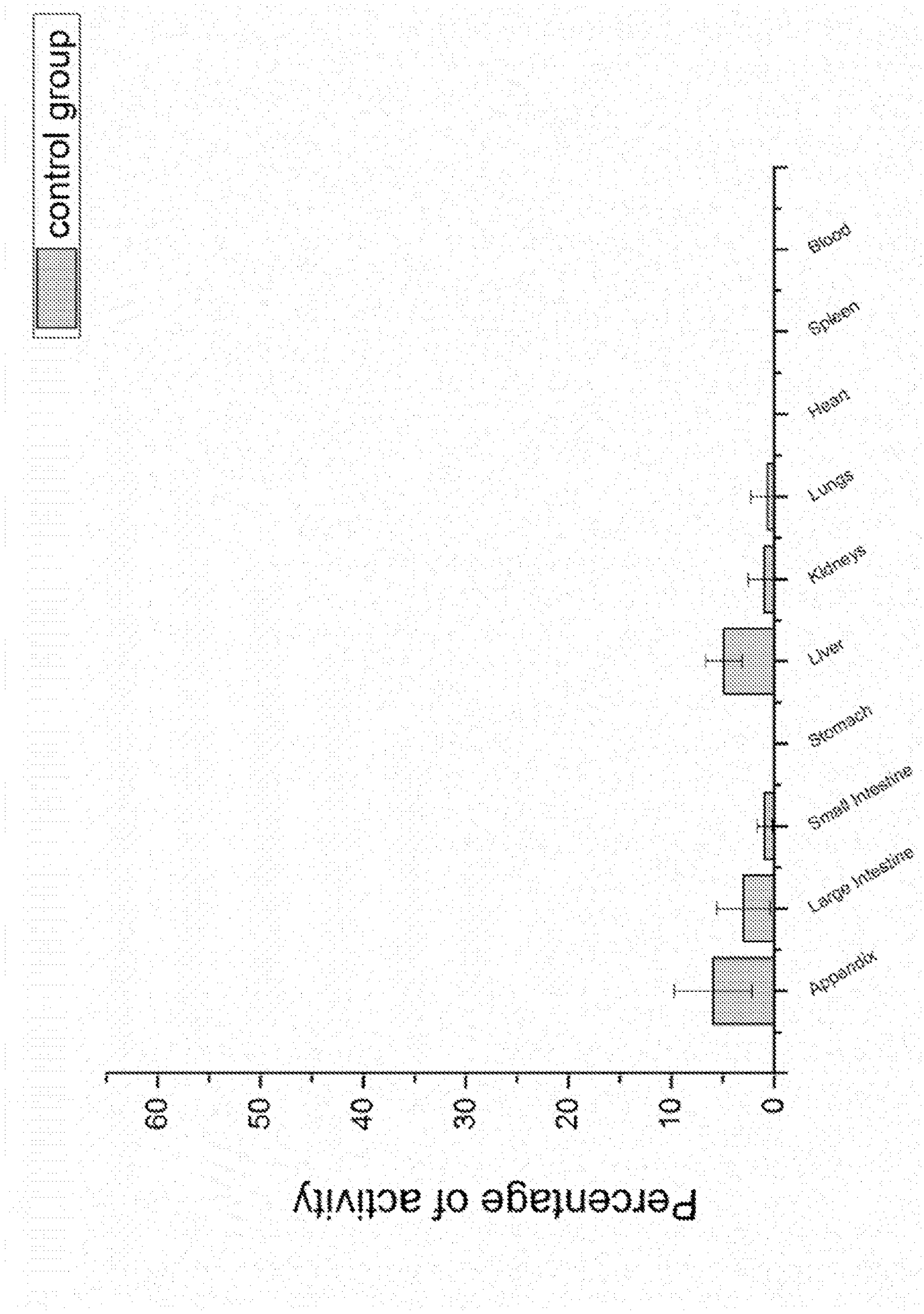
control group
Percentage of activity
0
10
20
30
40
50
60
Appendix
Large Intestine
Small Intestine
Stomach
Liver
Kidneys
Lungs
Heart
Spleen
Blood

Figure 5d

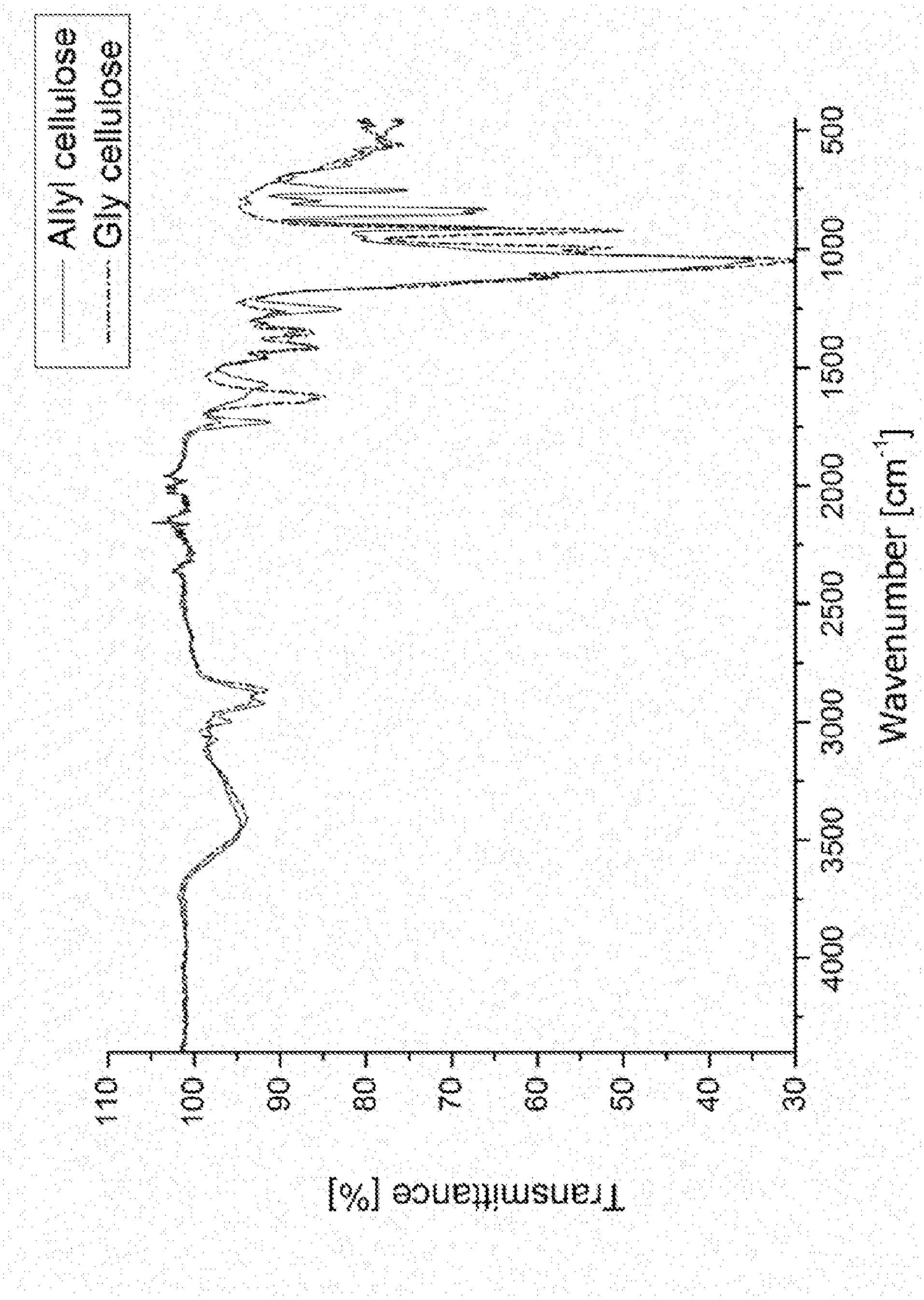
Allyl cellulose
Gly cellulose
Transmittance [%]
110
100
90
80
70
60
50
40
30
4000
3500
3000
2500
2000
1500
1000
500
Wavenumber [cm-1]

Figure 6

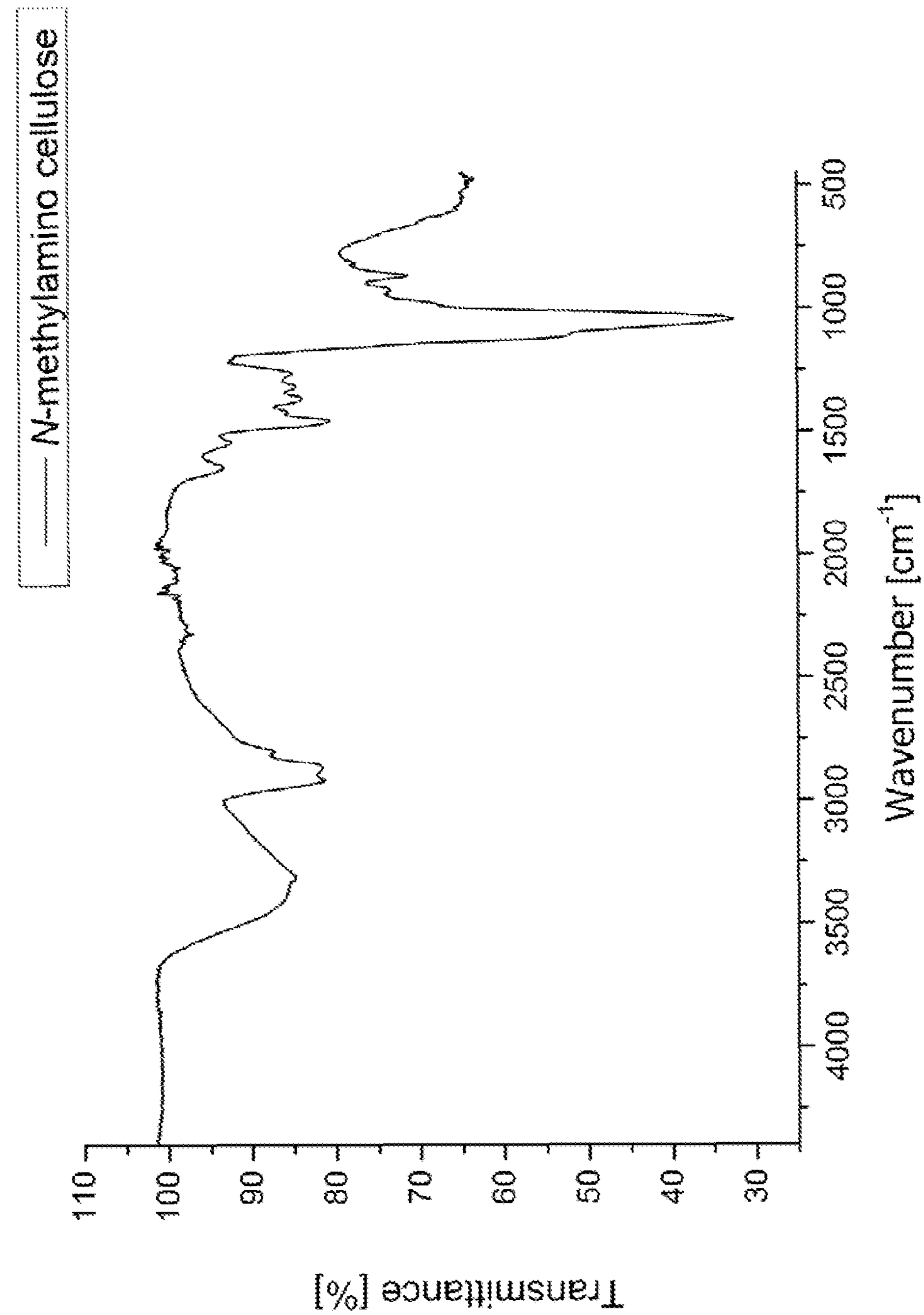
N-methylamino cellulose
Transmittance [%]
110
100
90
80
70
60
50
40
30
Wavenumber [cm-1]
4000
3500
3000
2500
2000
1500
1000
500

Figure 7

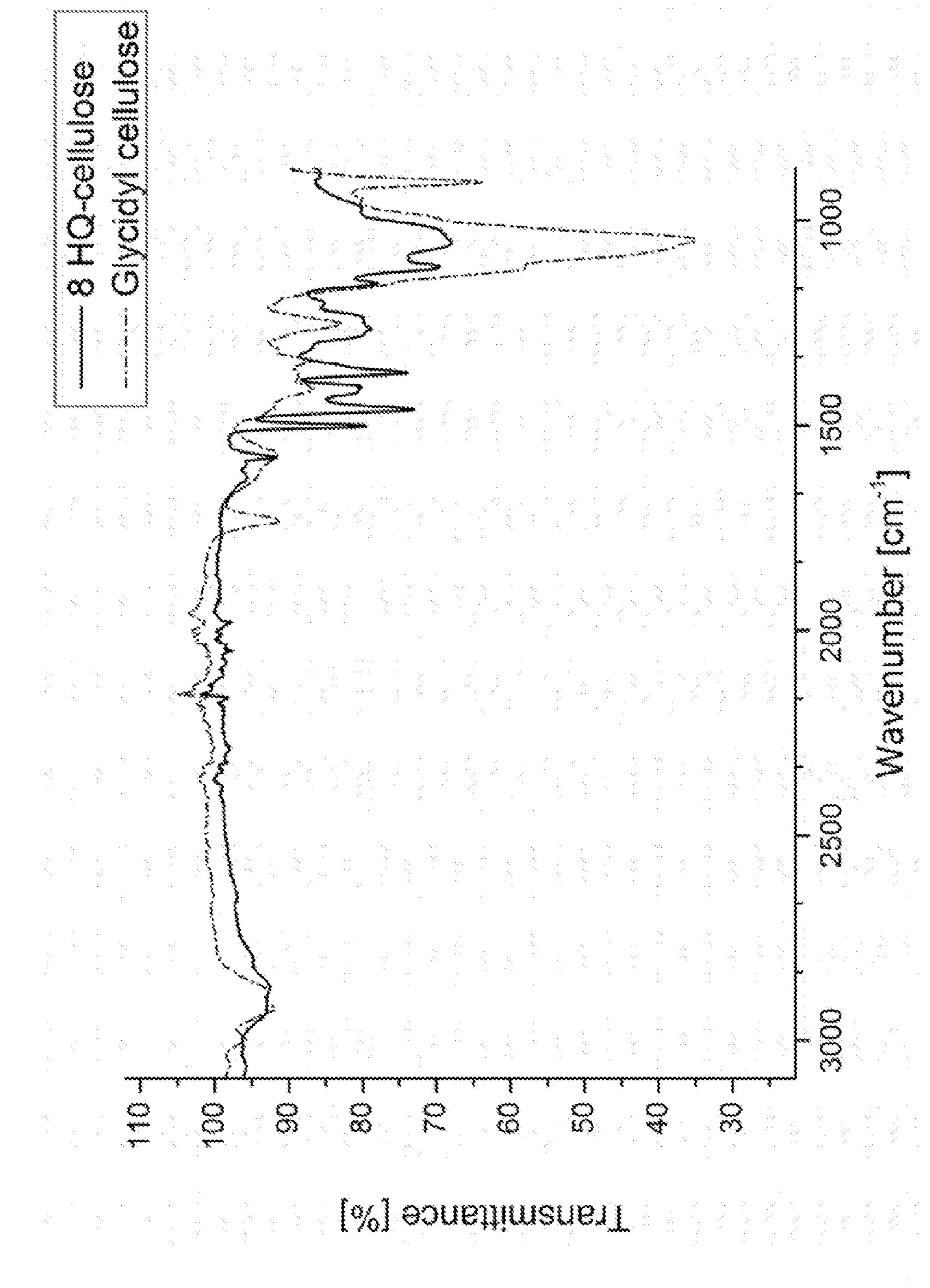
8 HQ-cellulose
Glycidyl cellulose
Transmittance [%]
Wavenumber [cm⁻¹]
110
100
90
80
70
60
50
40
30
3000
2500
2000
1500
1000

Figure 8

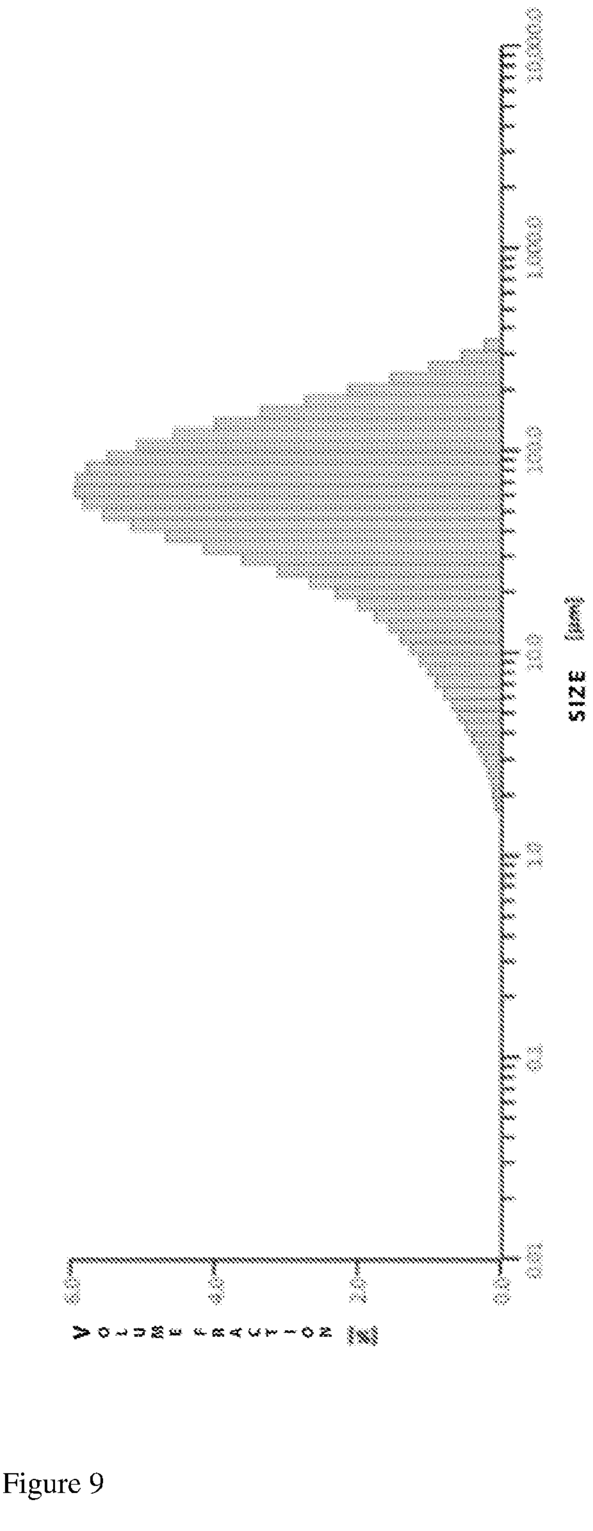


Figure 9

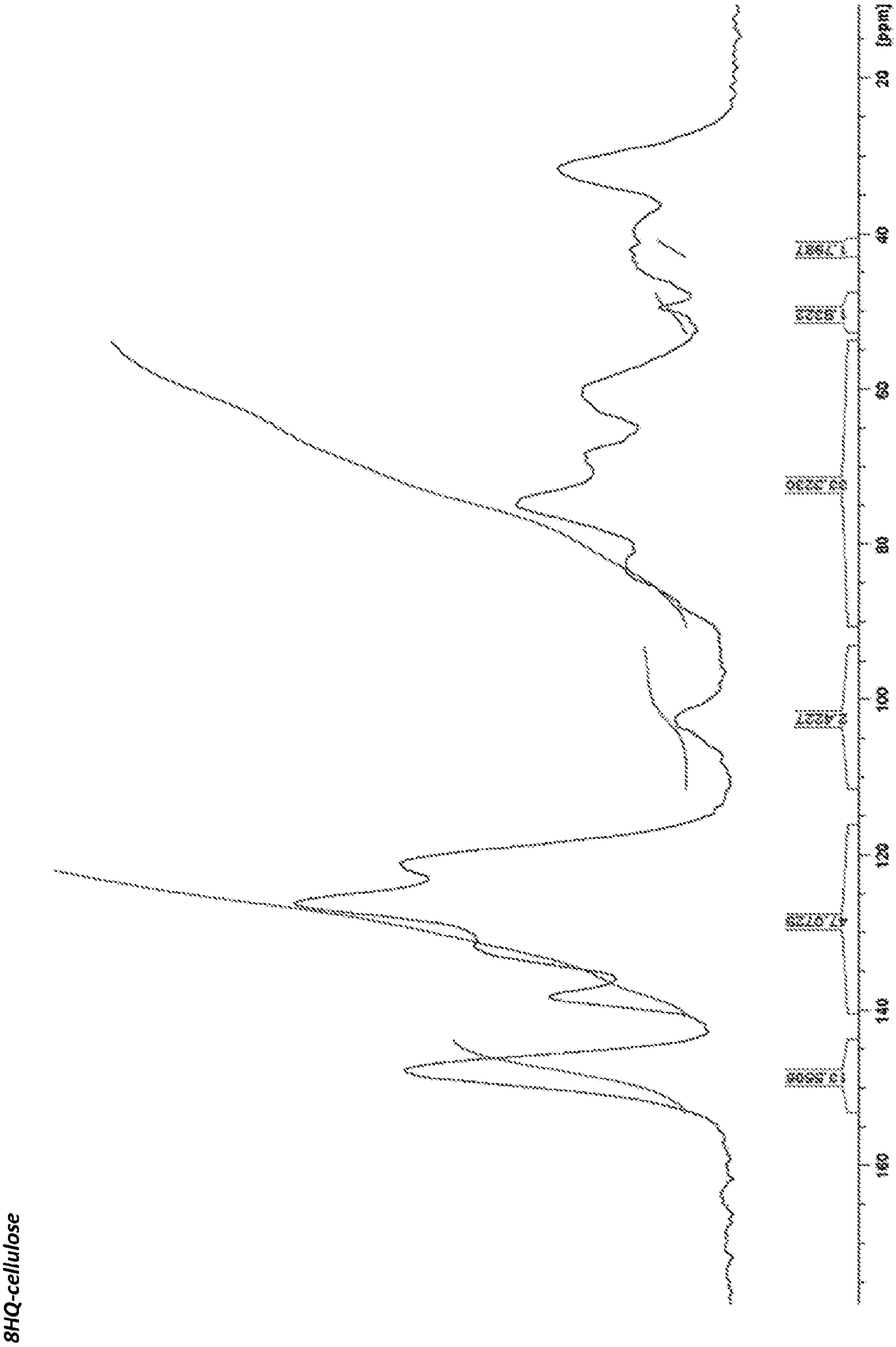


Figure 10

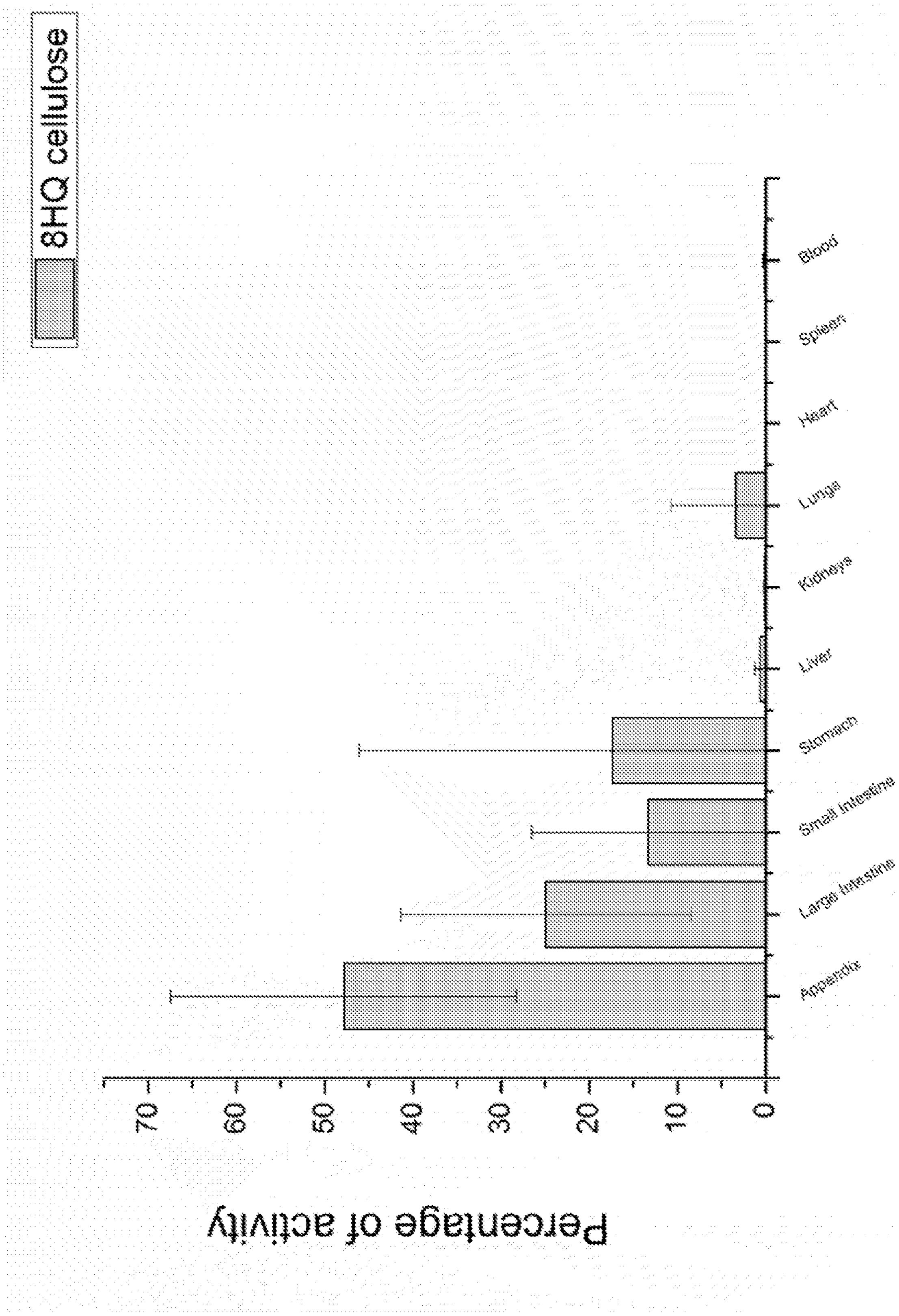
8HQ cellulose
Percentage of activity
0
10
20
30
40
50
60
70
Appendix
Large Intestine
Small Intestine
Stomach
Liver
Kidneys
Lungs
Heart
Spleen
Blood

Figure 11a

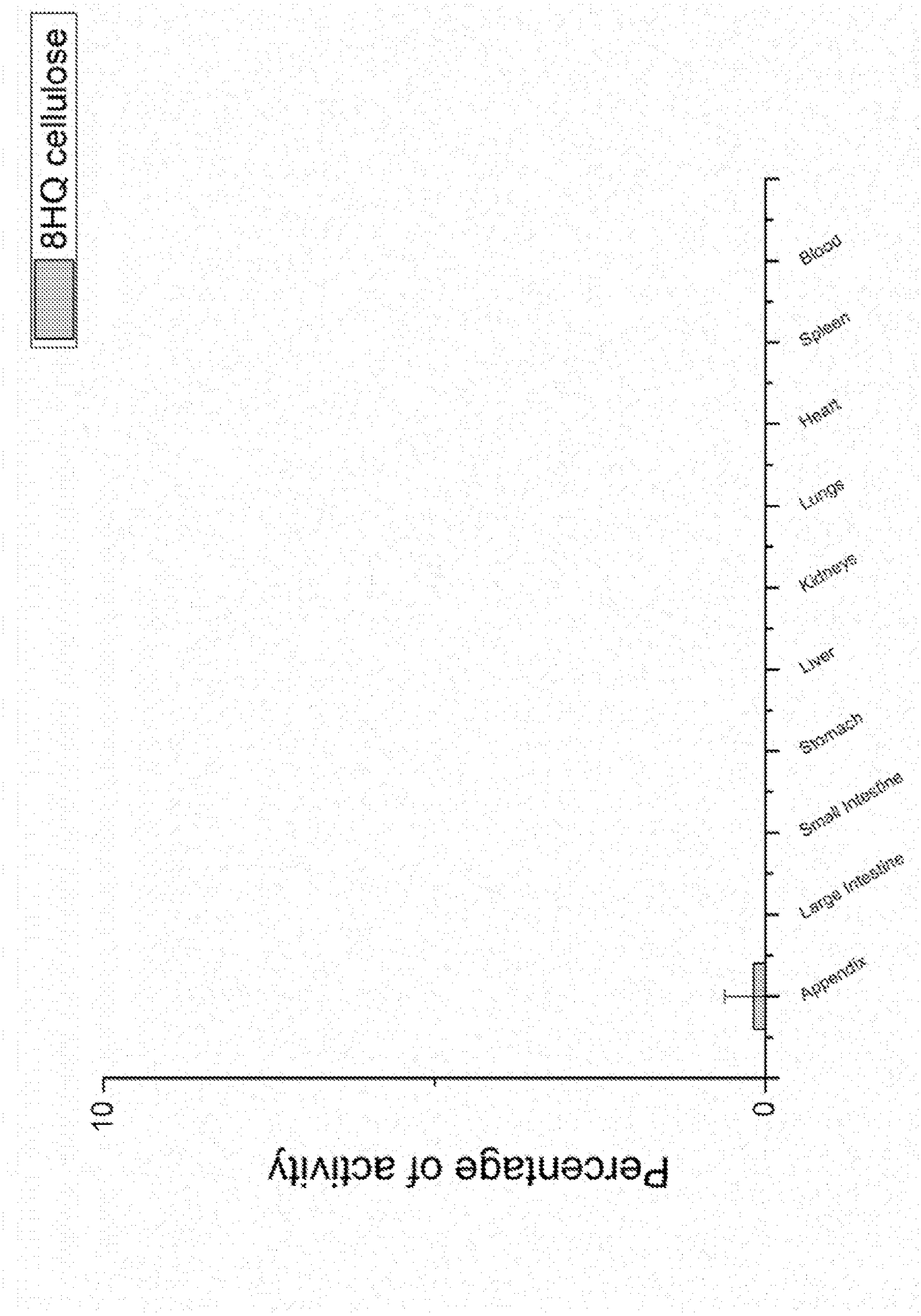
8HQ cellulose
Percentage of activity
10
0
Blood
Spleen
Heart
Lungs
Kidneys
Liver
Stomach
Small Intestine
Large Intestine
Appendix

Figure 11b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ALA, A. ; WALKER, A. P. ; ASHKAN, K. ; DOOLEY, J. S. ; SCHILSKY, M. L.** *Lancet,* 2007, vol. 369, 397-408
- **BRUHA, R. ; MAREK, Z. ; POSPÍŠILOVÁ, L. ; NEVŠÍMALOVÁ, S. ; VÍTEK, L. ; MARTÁSEK, P. ; NEVORAL, J. ; PETRTYL, J. ; URBÁNEK, P. ; JIRÁSKOVÁ, A.** Long-term follow-up of Wilson's Disease: natural history, treatment, mutations analysis and phenotypic correlation. *Liver International,* 2010, vol. 31, 83-91
- **HUSTER, D.** *Best Practice & Research in Clinical Gastroenterology,* 2010, vol. 24, 531-539
- **DAS, S. K. ; RAY, K.** *Nature Clinical Practice Neurology,* 2006, vol. 2, 482-493
- **BREWER, G. J. ; YUZBASIYAN-GURKAN, V. ; JOHNSON, V. ; DICK, R.D. ; WANG, Y.** Treatment of Wilson's Disease with Zinc XII: Dose Regimen Requirements. *The American Journal of the Medical Sciences,* 1993, vol. 305, 199-202
- **VAN DEN BERGHE, P. V. E. ; KLOMP, L. W. J.** New developments in the regulation of intestinal copper absorption. *Nutrition Reviews,* 2009, vol. 67, 658-672